# EUROPÄISCHE PATENTSCHRIFT

(11) **EP 3 097 876 B1**
(45) Veröffentlichungstag und Bekanntmachung des Hinweises auf die Patenterteilung: **17.03.2021**
(21) Anmeldenummer: 15169900.6
(22) Anmeldetag: 29.05.2015
(51) Int. Cl.: A61B 17/3203

(54) **DÜSENELEMENT ZUM AUFSTRAHLEN EINES WASSERSTRAHLS**
NOZZLE ELEMENT FOR PROJECTING A WATER JET
ÉLEMENT DE BUSE POUR PROJETER UN JET D'EAU

(43) Veröffentlichungstag der Anmeldung: 30.11.2016
(73) Patentinhaber: MEDAXIS AG, 6340 Baar (CH)
(72) Erfinder: Moser, Beat, 8926 Uerzlikon (CH); Zweifel, Adrian, 8645 Jona (CH); Widmer, Beat, 6005 Luzern (CH); Widmer, Matthias, 6300 Zug (CH)
(74) Vertreter: Grünecker Patent- und Rechtsanwälte PartG mbB

(56) Entgegenhaltungen:
- EP-A1- 2 251 142
- WO-A1-2015/032866
- WO-A2-2004/014460
- DE-A1- 19 928 441
- DE-U1-202004 014 104

## Beschreibung

Die vorliegende Erfindung betrifft ein Düsenelement zum Aufstrahlen eines Wasserstrahls mit einem Wasserdruck von zwischen 50 und 200 bar.

Seit langem sind Hochdruckreinigungsgeräte für das Aufstrahlen von Wasser auf eine zu behandelnde Oberfläche bekannt. Solche Verfahren werden üblicherweise in der Industrie und im Haushalt mit Hochdruckreinigungsgeräten durchgeführt, die einen mit einem Betätigungsknopf versehenen Handgriff aufweisen, mit dem der Hochdruckstrahl an- und ausgeschaltet werden kann. Der Hochdruckstrahl wird möglichst flächig auf die zu behandelnde Oberfläche aufgebracht. Dabei besteht ein Zielkonflikt zwischen einem fokussierten harten Strahl und einer angemessenen Größe des Strahles, um beim einmaligen Überstreichen der zu behandelnden Oberfläche den gewünschten Erfolg zu erzielen. Ein medizinisches Instrument entsprechend dem Obergriff des Anspruchs 1 ist aus der EP 2251142 A1 bekannt.

Diese dem Anmeldungsgegenstand zugrundeliegende Aufgabe wird durch die im Ansprüche 1 und 11 angegebenen Merkmale gelöst, weitere Ausführungsformen sind in den Unteransprüchen aufgeführt.

Die vorliegende Erfindung will ein neues Düsenelement zum Aufstrahlen eines Wasserstrahls zur Behandlung einer Oberfläche angehen. Dabei lässt sich die vorliegende Erfindung von der Vorstellung leiten, dass auch sensible Oberflächen mittels Wasserstrahl behandelt werden müssen, beispielsweise um organische Oberflächen zu behandeln, Gewebe zu reinigen und darauf vorgesehene Verkrustung zu lösen und abzutragen. Dies kann aus kosmetischen oder medizinischen Gründen zum Einsatz kommen. Das Düsenelement soll insbesondere beim Debridement zum Einsatz kommen.

Des Weiteren will die vorliegende Erfindung eine für die Durchführung des Verfahrens geeignete Vorrichtung angeben.

Zur Lösung des verfahrensmäßigen Problems wird Beispielsverfahren zum Aufstrahlen eines Wasserstrahls auf eine zu behandelnde Oberfläche mit den Merkmalen von Anspruch 1 angegeben. Das Aufstrahlen erfolgt dabei mit einem Wasserdruck von zwischen 50 und 200 bar, vorzugsweise mit zwischen 60 und 130 bar. Dieser Wasserduck wird üblicherweise pumpenseitig festgelegt. Bei dem Verfahren wird der Wasserstrahl durch ein Düsenelement abgegeben, das einen zylindrischen Düsenkörper hat. Dieser bildet einen Strömungsdurchgang für den Wasserstrahl aus. Des Weiteren hat das Düsenelement eine Düsenöffnung, die den Düsenstrahl an die Umgebung abgibt. Der zuvor erwähnte Wasserdruck liegt üblicherweise auch als Staudruck innerhalb des Düsenkörpers und in Strömungsrichtung unmittelbar vor der Düsenöffnung an. Zur Durchführung des Verfahrens ist die Düsenöffnung verhältnismäßig klein gewählt, um den Wasserstrahl mit einem überschaubaren Strahlstoßdruck auf die zu behandelnde Oberfläche aufzubringen. Die kleinste Abmessung der Düsenöffnung ist bei dem Verfahren nicht mehr als 0,15 mm, vorzugsweise nicht mehr als 0,13 mm und mindestens 0,02 mm groß. Als kleinste Abmessung wird dabei im Falle einer kreisrunden Öffnung der Durchmesser, im Falle einer ovalen Öffnung der kleinste Durchmesser und im Falle einer länglichen Düsenöffnung die Breite derselben angesehen. Die Düsenöffnung hat dabei vorzugsweise eine Öffnungsfläche von nicht mehr als 1,8·10⁻² mm² bevorzugt zwischen 4,6·10⁻³ mm² und 1,3·10⁻² mm². Die Düsenöffnung, gegebenenfalls auch die Geometrie des der Düsenöffnung vorgelagerten Strömungsdurchgangs ist dabei so gewählt, dass in einem Abstand von 80 mm zwischen der Düsenöffnung und der zu behandelnden Oberfläche auf der zu behandelnden Oberfläche ein Flachstrahl ausgebildet wird. Als Flachstrahl im Sinne der vorliegenden Erfindung wird ein Strahl verstanden, dessen Längen-zu-Breiten-Verhältnis zumindest 2,5 beträgt. Üblicherweise liegt das Verhältnis von Länge zu Breite des Flachstrahles bei zwischen 2,5 und 15, bevorzugt zwischen 4 und 10. Dieses Längenverhältnis wird von dem für die Behandlung notwendigen Strahlstoßdruckbereich des auf die Oberfläche austreffenden Flachstrahles erfüllt. Zu diesem Längenverhältnis tragen keine Tropfen oder Nebel bei, die durch Oberflächeneffekte an dem Strahl von der Oberfläche des Strahls herausgelöst und mit nur zu geringem Impuls auf die zu reinigende Oberfläche in dem besagten Arbeitsabstand auftreffen.

Mit dem Verfahren ist es möglich, verhältnismäßig sensible Oberflächen mit einem Wasserstrahl zu reinigen. Der Wasserstrahl kann mit Reinigungszusätzen versehen sein. Solche Reinigungszusätze können auch antibakterielle Zusätze sein, mit denen Bakterien auf der zu reinigenden Oberfläche abgetötet werden. Gegebenenfalls kann ergänzend auch ein Lichtstrahl aufgebracht werden, der Wellen in einem keimtötenden Bereich aussendet und üblicherweise im Wesentlichen parallel zu dem Wasserstrahl abgegeben wird, bevorzugt von einem Handstück ausgesandt wird, welches das Düsenelement in sich aufnimmt und gleichzeitig eine Lichtausgabeöffnung umfasst. Die Lichtquelle kann dabei in dem Handstück vorgesehen sein. Alternativ kann das ausgesendete Licht auch über einen Lichtleiter dem Handstück zugeführt werden. Die Lichtquelle sendet dabei vorzugsweise Licht mit einer Wellenlänge im ultravioletten Bereich aus. Eine solche Verfahrensführung eignet sich beispielsweise für die kosmetische Behandlung von Haut, um Unreinheiten abzulösen und gegebenenfalls auch Bakterien abzutöten.

Gemäß einer bevorzugten Weiterbildung der vorliegenden Erfindung wird in dem Arbeitsabstand von 80 mm auf einem Flächenanteil von zumindest 3 mm² der zu behandelnden Oberfläche ein Strahlstoßdruck von zumindest 1.000 Pa und bevorzugt von nicht mehr als 60.000 Pa erzeugt. Oberhalb des angegebenen oberen Grenzwertes für den Strahlstoßdruck wird bei dem Verfahren eine nicht zu akzeptierende Beeinträchtigung der zu behandelnden Oberfläche befürchtet. Unterhalb des besagten Strahlstoßdrucks wird der durch die Behandlung zu erzielende Effekt nicht mehr in dem gewünschten Maß erreicht. Der angegebene Flächenanteil entspricht dabei dem Bedürfnis einer hinreichend großflächigen Reinigung der zu reinigenden Oberfläche. Der Strahlstoßdruck ist dabei derjenige Druck, der aus der Komponente der Impulskraft rechtwinklig zu der behandelnden Oberfläche auftritt und gemessen wird. Bevorzugte höhere Untergrenzen für den innerhalb Flächenanteil wirkenden Druck sind 5.000 Pa bzw. 10.000 Pa Bevorzugte tiefere Obergrenzen für den innerhalb Flächenanteil wirkenden Druck sind 40.000 Pa und 35.000 Pa. Der Flächenanteil sollte mit Blick auf eine überschaubare zu behandelnde Fläche bevorzugt nicht größer als 100 mm², besonders bevorzugt nicht größer als 50 mm², ganz besonders bevorzugt nicht größer als 20 mm², und noch bevorzugter nicht größer als 12 mm² sein.

Praktische Versuche der Anmelderin haben ergeben, dass sich der Flachstrahl auf dem Weg zwischen der Düsenöffnung und der zu behandelnden Oberfläche mitunter aufteilt und beispielsweise zwei gegebenenfalls auch mehrere Bereiche mit einem relativ hohen Strahlstoßdruck umfasst, die zwischen sich Bereiche mit geringem Strahlstoßdruck einschließen, wobei dieser Strahlstoßdruck keine hinreichende Wirkung an der zu behandelnden Oberfläche zeigt. Dabei wird die Wirkung durch den Strahl auf der Oberfläche jedenfalls dann nicht nachteilig beeinflusst, wenn der für eine hinreichende Wirkung als notwendig erachtete Strahlstoßdruck sich außen um einen Bereich des Strahlstoßdruckprofils auf der zu behandelnden Oberfläche befindet, der einen Bereich mit niedrigeren Strahlstoßdruckwerten in sich einschließt. So wird gemäß einer bevorzugten Weiterbildung der vorliegenden Erfindung und mit Blick auf eine wirtschaftliche Behandlung der Oberfläche vorgeschlagen, auf der zu behandelnden Oberfläche ein Strahlstoßprofil zu erzeugen, das eine homogene, kontinuierliche, längliche und vorzugsweise linienförmige Ausbildung hat.

Praktische Versuche haben ferner gezeigt, dass es zur Ausbildung eines Flachstrahls mittels eines Sprühmediums, welches eine ähnliche Viskosität und Oberflächenspannung wie Wasser in einem Temperaturbereich zwischen 20 und 40 °C besitzt, günstig ist, innerhalb des Düsenkörpers, d.h. in dem Strömungsdurchgang und der Düsenöffnung bestimmte Reynoldszahlen für die Strömung einzuhalten. Der in den Unteransprüchen insofern gemachte Vorschlag orientiert sich an einem Druckbereich zwischen 50 und 200 bar für den Wasserdruck bei Raumtemperatur.

Wird in diesem bevorzugten Druck- und Temperaturbereich eine Strahlbehandlung der Oberfläche mittels eines geeigneten Sprühmediums unter Einfluss der Atmosphäre erzeugt, so lässt sich mit guter Genauigkeit ein Flachstrahl mit der gewünschten Zerstäubung erzeugen. So wird mit der vorliegenden Erfindung vorgeschlagen, die Reynoldszahl in dem Strömungsdurchgang eingangsseitig zwischen 450 und 28.000 und ausgangsseitig mit etwa 3.900 bis 46.000 festzulegen. Die entsprechenden Reynoldszahlen lassen sich durch fluiddynamische Analysen anhand der Geometrie und den entsprechenden Randbedingungen vorausberechnen. Für die Erzeugung der Auffächerung eines Strahls zu einem Flachstrahl ist die radiale Geschwindigkeitskomponente am Düsenaustritt maßgebend. Die Anmelderin hat bei der Vorausberechnung der Strömungseigenschaften, die bei Verfahren wirken, nummerische Strömungsberechnungen durchgeführt und dabei festgestellt, dass insbesondere für Freistrahlsimulationen die Anisotropie der Turbulenz eine wichtige Rolle spielt. Für diese Berechnungen zeigte sich das Reynolds-Stress-Turbulenzmodell BSL von Ansys CFX 14.5 hilfreich. Das Modell als solches ist beispielsweise beschrieben in (ANSYS ® Academic Research, Release 14.5, Help System, Solver Modeling Guide, ANSYS, Inc.). Für die Strömung im Düsenkörper und in der Düsenöffnung wurde das SST Turbulenzmodell von Ansys CFX 14.5 verwendet. Mit Hilfe dieses Modells lässt sich zuverlässig die radiale Geschwindigkeitskomponente ermitteln, anhand welcher das Strahlbild bestimmt werden kann. Mittels geeigneter Modelle lassen sich auch Zerstäubungsvorgänge erfassen, die den kompakten Strahl auflösen und selbst bei einem relativ geringen Arbeitsabstand von 80 mm den gewünschten Strahlstoßdruck erzeugen.

Nach den derzeitigen Überlegungen sind auch die Strömungsbedingungen von Bedeutung, die in Strömungsrichtung vor dem Düsenkörper eingestellt werden. Der Düsenkörper hat üblicherweise eine Länge von zwischen 1 und 30 mm, bevorzugt von zwischen 1 und 10 mm. Der Durchmesser des üblicherweise mit rundem Querschnitt vorgesehenen Strömungsdurchgangs kann dabei zwischen 0,15 und 0,6 mm, bevorzugt zwischen 0,25 und 0,35 mm, besonders bevorzugt bei 0,3 ± 0,05 mm liegen. Dieser Düsenkörper wird üblicherweise in einem Handstück aufgenommen, der einen zu dem Strömungsdurchgang führenden Zwischenkanal ausbildet.

Der Übergang zwischen dem Strömungsdurchgang und der Düsenöffnung ist vorzugsweise unstetig. So haben Versuche der Anmelderin ergeben, dass eine bevorzugte Beeinflussung des Strahls als Flachstrahl dadurch möglich ist, dass die Düsenöffnung streng zylindrisch ausgebildet ist. Auch der der Düsenöffnung vorgelagerte Strömungsdurchgang ist vorzugsweise streng zylindrisch ausgebildet. Die Düsenöffnung ist dementsprechend in einer den Strömungsdurchgang abschließenden Blende vorgesehen. Vorzugsweise ist die Düsenöffnung konzentrisch zu dem Strömungsdurchgang vorgesehen. Eine solche Ausgestaltung erlaubt auch eine kostengünstige Herstellung des zur Durchführung des Verfahrens mit dem nebengeordneten der vorliegenden Erfindung vorgeschlagenen Düsenelements. Das erfindungsgemäße Düsenelement ist in Anspruch 1 angegeben.

Soweit vorstehend auf die Verfahrensdurchführung und die besonderen Bedingungen innerhalb des Düsenelementes sowie die Eigenschaften des Flachstrahles abgestellt worden sind, gelten diese Weiterbildungen in gleicher Weise für den Düsenkörper. Wenn gleich das Verfahren innerhalb des zunächst vorstehend genannten Druckintervalls von zwischen 50 und 200 bar durchgeführt werden kann, ist ein Druckbereich von zwischen 50 und 130 bar für die Verfahrensdurchführung zu bevorzugen.

Gemäß einer bevorzugten Weiterbildung der vorliegenden Erfindung wird der Strahl durch eine als Breitschlitzdüse ausgebildete Düsenöffnung abgegeben und als Flachstrahl mit einer in etwa rechtwinklig zu der Längserstreckung der Breitschlitzdüse versetzten Ausrichtung auf die zu behandelnde Oberfläche aufgebracht. In der Luftstrecke zwischen der Düsenöffnung der zu behandelnden Oberfläche ergibt sich somit eine Umorientierung des Strahles.

Für die Charakterisierung des erfindungsgemäßen Düsenelementes hingegen wird auf einen Wasserdruck von 130 bar abgestellt. Düsenelemente, die bei einem solchen Systemdruck respektive einem solchen Druck innerhalb des Strömungsdurchgangs die zuvor diskutierten Strahlstoßdruckwerte (minimale und maximale) sowie Strahlstoßdruckprofile auf der zu behandelnden Oberfläche bei dem genannten Arbeitsabstand von 80 mm zeigen, sind Weiterbildungen des erfindungsgemäßen Düsenelementes. Es hat sich gezeigt, dass eine Vielzahl von Düsenelementen mittels fluiddynamischer Vorausberechnung und Analyse geschaffen werden können. So definiert die vorliegende Erfindung das erfindungsgemäße Düsenelement vornehmlich über die Parameter, die den erzeugten Flachstrahl unter den genannten Bedingungen (130 bar Wasserdruck, Arbeitsabstand 80 mm) charakterisieren.

Das Düsenelement der vorliegenden Erfindung ist danach bevorzugt so ausgebildet, dass es bei einem Arbeitsdruck von 130 bar und einem Arbeitsabstand zwischen der Düsenöffnung und der zu behandelnden Oberfläche von 80 mm auf der zu behandelnden Oberfläche einen Flachstrahl mit den Merkmalen der Ansprüche 1, 2 und 6 ausbildet. Das Düsenelement wird vorzugsweise verwendet zum Debridement und so ist auch die Verwendung des hier spezifizierten Düsenelementes für das Debridement beansprucht. Die erfindungsgemäße Düse ist dabei weitaus einfacher und damit kostengünstiger herzustellen als beispielsweise die aus der EP 2 251 142 A1 vorbekannte Düse. Im Übrigen erzeugt diese vorbekannte Düse keinen Flachstrahl.

Der unstetige Übergang zwischen der Düsenöffnung und dem Strömungsdurchgang lässt sich fertigungstechnisch dadurch recht einfach erzielen, dass der Düsenkörper aus Metall, insbesondere Stahl gebildet ist und dass das Düsenblech mit dem Düsenkörper verschweißt ist. Der Düsenkörper sowie das Düsenblech sind üblicherweise stoffidentisch ausgebildet. Die Düsenöffnung kann mittels Laser geschnitten oder fotolithografisch oder mittels Ätzen ausgebildet sein. Das Düsenelement mag ein Verbrauchsteil sein, sodass das Düsenelement nach einer einmaligen Benutzung verworfen wird. Dies ist insbesondere dann von Bedeutung, wenn es auf hygienische Randbedingungen ankommt bzw. das Düsenelement und/oder das Handstück bei der ersten Benutzung kontaminiert wird und nicht wiederverwendet werden kann. Hygienische Bedingungen können auch bei der Behandlung von Biooberflächen bedeutsam sein. So sollte der Düsenkörper kostengünstig herzustellen sein, was mit der genannten Verschweißung eines im Grund zylindrischen Düsenkörpers mit einer Lochblende als Düsenblech gewährleistet ist. Die beiden Bauteile werden üblicherweise durch stirnseitiges Laserschweißen gegen die Oberfläche des Düsenblechs miteinander verschweißt. Eine solche Verschweißung führt zu einer fluiddichten Verbindung zwischen dem Düsenblech und dem Düsenkörper.

Wie bereits erwähnt, ist die Düsenöffnung vorzugsweise als Breitschlitzdüse ausgebildet. Das Verhältnis von Länge der Breitschlitzdüse zu Breite der Breitschlitzdüsen, d.h. das Länge-zu-Breiten-Verhältnis sollte bei zwischen 1 und 7 bevorzugt zwischen 3 und 4 liegen. Als Länge wird dabei die größte Erstreckung der Breitschlitzdüse verstanden. Die Breite ist die Erstreckung in einer Richtung rechtwinklig hierzu jeweils in einer Ebene, die orthogonal von der Strömung innerhalb der Düse durchsetzt ist. Mit anderen Worten das Verhältnis die Ausgestaltung des Düsenquerschnitts. Die Breite der Breitschlitzdüse liegt dabei zwischen 0,035 und 0,06 mm, bevorzugt zwischen 0,04 und 0,055 mm.

Die Anmelderin hat systematische Untersuchungen zu verschiedenen Düsengeometrien durchgeführt. Dabei wurden kreuzschlitzförmige Düsen und auch Düsen mit einer breiten, indes nicht gradlinig verlaufenden Querschnittform untersucht. Auch wurden elliptische Düsen sowie Düsenbleche mit mehreren beispielsweise in einer Reihe nebeneinander vorgesehenen Bohrungen untersucht. Es hat sich herausgestellt, dass die Strömungsbedingungen, die zuvor diskutiert wurden sind, zumindest ebenso bedeutsam für die Ausbildung eines relativ kleinen Flachstrahles in dem gewünschten Arbeitsabstand sind, wie die geometrische Ausgestaltung der Düse.

Es hat sich ferner ergeben, dass eine Breitschlitzdüse, deren Düsenöffnung gradlinig verlaufende Hauptseitenwände und konkav gekrümmte Seitenwände, deren Krummungsradius zumindest der halben Breite der Düsenöffnung entspricht und der nicht größer als 0,15 mm ist, aufweist einen sehr guten Flachstrahl erzeugt. Bei dieser Ausgestaltung erstrecken sich die Hauptseitenwände parallel und gradlinig zueinander. Sie enden mit konkaver Krümmung. Der Radius der Krümmung ist minimal die halbe Breite der Breitschlitzdüse. Maximal beträgt der Radius 0,15 mm. Die Düsenöffnung sollte mit Blick auf die bevorzugten Strömungsbedingungen regelmäßig versetzt zu der Innenumfangsfläche des Strömungsdurchgangs vorgesehen sein, sodass die gewünschte Unstetigkeit zwischen dem Strömungsdurchgang und der Düsenöffnung gegeben ist.

Es hat sich überraschenderweise auch gezeigt, dass mit einer kreisrunden Düsenöffnung mit einem Durchmesser von zwischen 0,09 und 0,12 mm ein Flachstrahl erzeugt werden kann. Entsprechendes ist auch möglich mit einem korrespondierenden mittleren ovalen Durchmesser, wobei der kleinste Durchmesser des Ovals bevorzugt zwischen 0,08 und 0,11 mm und der größere Durchmesser des Ovals nicht mehr als 1,3-fach größer als der kleinste Durchmesser sein sollte. Dabei lässt sich der Flachstrahl bevorzugt dadurch ausbilden, dass dem Düsenblech in Strömungsrichtung zumindest ein Blendenblech vorgelagert ist, dessen Blendenöffnung größer als die Düsenöffnung ist. Die Blendenöffnung sollte indes eine Düsenfläche von nicht mehr als 150% der Düsenfläche der Düsenöffnung haben. Im Falle einer rotationssymmetrischen Düsenöffnung sollte die Blendenöffnung nicht rotationssymmetrisch, allerdings konzentrisch zu dem Mittelpunkt der Düsenöffnung ausgebildet und angeordnet sein. Auch die Blendenöffnung ist vorzugsweise streng zylindrisch, sodass sich die die Blendenöffnung definierenden Wandungen rechtwinklig zu den Innen- bzw. Außenflächen des die Blende ausformenden Blendenblechs erstrecken. Bei einer kreisrunden Düsenöffnung kann die Blendenöffnung beispielsweise oval oder als Langloch ausgeformt sein, wobei das Langloch nur geringfügig breiter als der Durchmesser der Blendenöffnung ist. Die Breite des Langloches sollte zwei bis fünf Hundertstelmillimeter größer sein als der Durchmesser der Düsenöffnung.

Gemäß einer weiteren bevorzugten Ausgestaltung der vorliegenden Erfindung sind zwei Blendenbleche in Strömungsrichtung dem Düsenblech vorgelagert vorgesehen. Wie bei der zuvor beschriebenen Ausgestaltung ist jedes einzelne Blech bevorzugt mittels Laserschweißen mit der darunterliegenden Lage verbunden. Das in Strömungsrichtung erste Blech ist danach mit dem Düsenkörper verschweißt, das in Strömungsrichtung darauffolgende Blech mit dem darunterliegenden Blech, usw. Die in Strömungsrichtung hintereinander vorgesehenen Blendenbleche haben üblicherweise voneinander abweichende Blendenöffnungen. Die Blendenöffnungen sind vorzugsweise nicht rotationssymmetrisch zu der Mittellängsachse der Düsenöffnung, d.h. dem Mittelpunkt einer kreisrunden Düsenöffnung vorgesehen, jedoch punktsymmetrisch zu diesem Punkt bzw. zu dieser Achse ausgeformt bzw. angeordnet. Hierdurch kann der Strömung beim Austritt aus dem Düsenelement ein Drall aufgeprägt werden. So kann das in Strömungsrichtung erste Blendenblech mit zwei oder mehreren einander gegenüberliegenden und im Grunde ringsegmentförmig ausgebildeten Blendenöffnungen versehen sein. Diesen beiden Blendenöffnungen kann ein zweites Blendenblech folgen, das eine mittlere kreisrunde Blendenöffnung hat, von dem Schlitze abgehen, die mit den ringsegmentförmigen Blendenöffnungen fluchten. Die jeweiligen Bleche sollten eine Dicke von zwischen 0,06 und 0,20 mm haben.

Mit der vorliegenden Erfindung wird ferner ein Schlauchelement mit einem Fluidschlauch, der an einem Eintrittsende mit einem Verbindungselement zum Anschluss des Schlauchelementes an eine Pumpe und an seinem Austrittsende mit einem Handstück versehen ist, vorgeschlagen. Das Handstück ist endseitig mit dem zuvor beschriebenen Düsenelement nach einem der zuvor diskutierten Ansprüche verbunden. Dieses Schlauchelement kann als verkehrsfähige Einheit vormontiert sein, was sich insbesondere dann empfiehlt, wenn das Düsenelement aufgrund seiner geringen Größe schwer zu handhaben ist. Das Düsenelement kann in das zuvor erwähnte Handstück eingeklebt sein. Alternativ kann das Düsenelement auch zwischen einem Handstückhauptkörper und einer Endkappe geklemmt sein, die lösbar mit dem Handstückhauptkörper verbunden ist, beispielsweise über einen Bajonett- oder Schraubverschluss. Dabei übergreift die Endkappe den Düsenkörper und drückt diesen vorzugsweise gegen eine Anlageschulter, die den Zwischenkanal strömungsausgangsseitig begrenzt.

Die vorliegende Erfindung schlägt ferner ein Handstück für ein solches Schlauchelement vor. Das Handstück zeichnet sich dadurch aus, dass es auch ohne den Fluidschlauch des Schlauchelementes hergestellt und vertrieben werden kann, um später nutzerseitig mit einem beliebigen Schlauchelement verbunden zu werden. Dazu hat das erfindungsgemäße Handstück einen Handstückhauptkörper, der eine zum Durchführen des Fluidschlauches geeignete längliche Bohrung aufweist. Der Handstückhauptkörper ist üblicherweise nach ergonomischen Gesichtspunkten geformt, sodass er zwischen den Fingern einer Hand gehalten werden kann. Der Handstückhauptkörper ist üblicherweise schlank, länglich und mit rotationssymmetrischem Querschnitt ausgebildet. Der Handstückhauptkörper trägt endseitig eine Endkappe. Diese ist üblicherweise lösbar mit dem Handstückhauptkörper verbunden. Die Endkappe schließt jedenfalls zwischen sich und dem Handstückhauptkörper ein Adapterstück ein. Diese Adapterstück ist üblicherweise aus Kunststoff ausgebildet und trägt das zuvor diskutierte Düsenelement, welches vorzugsweise aus Metall ausgebildet ist. Dieses Düsenelement ist üblicherweise mit dem Adapterstück verklebt. Das Adapterstück hat regelmäßig eine Bohrung zur Aufnahme des Düsenelementes. Desweiteren sind regelmäßig Anschlussmittel zum dichtenden Anschluss des Fluidschlauches an das Adapterstück vorgesehen. Bei einer einfachen Ausgestaltung hat das Adapterstück eine Bohrung, in welche der Fluidschlauch mit seinem freien Ende eingeführt und dort fluiddicht mit dem Adapterstück verbunden werden kann. Bei einer einfachen Ausgestaltung nach Art eines Einwegproduktes wird auch der Fluidschlauch mit dem Adapterstück verklebt.

Dabei ist das Düsenelement vorzugsweise so in dem Adapterstück aufgenommen, dass das Düsenelement mit seinem freien Ende das Adapterstück überragt. In entsprechender Weise überragt das Düsenelement üblicherweise auch die das Düsenelement stirnseitig übergreifende Endkappe, sodass das Düsenelement gegenüber dieser Düsenkappe geringfügig vorsteht. Die Endkappe hat üblicherweise eine Bohrung, die von dem Düsenelement durchsetzt ist. Die Bohrung lässt aber üblicherweise einen hinreichenden radialen Abstand zu dem vorderen Ende des Düsenelementes. Es kommt vornehmlich darauf an, dass die Endkappe das Adapterstück endseitig übergreift und in axialer Richtung durch üblicherweise verschrauben der Endkappe gegen den Handstückhauptkörper das Adapterstück in axialer Richtung in dem Handstück fixiert.

Die vorliegende Erfindung betrifft schließlich ein Verfahren zur Herstellung eines Düsenkörpers, bei dem zumindest eine Düsenöffnung an einem Düsenblech mittels Laserschweißen ausgebildet wird. Das Düsenblech wird dabei aus einer Düsenblechplatte geschnitten, wobei Verbindungsstege zwischen dem Düsenblech, welches mit dem Düsenkörper mittels Laserschweißen verbunden wird, und dem Blechstück verbleiben. So kann das relativ kleine Düsenblech über das Blechstück gehandhabt und positioniert werden. Danach wird das Düsenblech mit dem Düsenkörper verschweißt. Dabeiwerden die verbleibenden Verbindungsstege durchtrennt, um das mit dem Düsenkörper verbundende Düsenblech von dem Halbzeug zu isolieren. Der zum Schweißen verwendete Strahl wird dementsprechend auch durch Schneiden der Verbindungsstege genutzt.

Die vorliegende Erfindung wird nachstehend anhand von Ausführungsbeispielen in Verbindung mit der Zeichnung näher erläutert. In dieser zeigen:
- Figur 1a und b: eine Längsseiten - bzw. Längsschnittansicht eines Ausführungsbei spiel eines Schlauchelementes;
- Figur 2: das in Figur 1b mit II gekennzeichnete Detail in vergrößerter Darstellung;
- Figur 3a: eine Längsschnittansicht eines ersten Ausführungsbeispiels eines Düsenelementes;
- Figur 3b: eine Draufsicht auf das vordere Düsenblech 20 nach Figur 3a;
- Figur 3c: eine Draufsicht auf das hintere Düsenblech 24 nach Figur 3a;
- Figur 3d: eine perspektivische stirnseitige Ansicht des ersten Ausführungsbeispiels;
- Figur 4a: eine Seitenansicht eines zweiten Ausführungsbeispiels eines Düsenelementes;
- Figuren 4b-4d: Draufsichten von Düsen- und Blendenblechen des zweiten Ausführungsbeispiels;
- Figur 4e: eine Draufsicht auf die hintereinander vorgesehenen Bleche der Figuren 4b-4d;
- Figur 5: eine perspektivische Ansicht eines weiteren Ausführungsbeispiel eines Düsenbleches mit Teilen des Düsenkörpers;
- Figur 6: eine perspektivische Draufsicht auf einen Flachstrahl, der von dem in Figur 5 gezeigten Blendenblech abgegeben wird; und
- Figuren 6a-6c: Profile des Flachstrahles nach Figur 6.

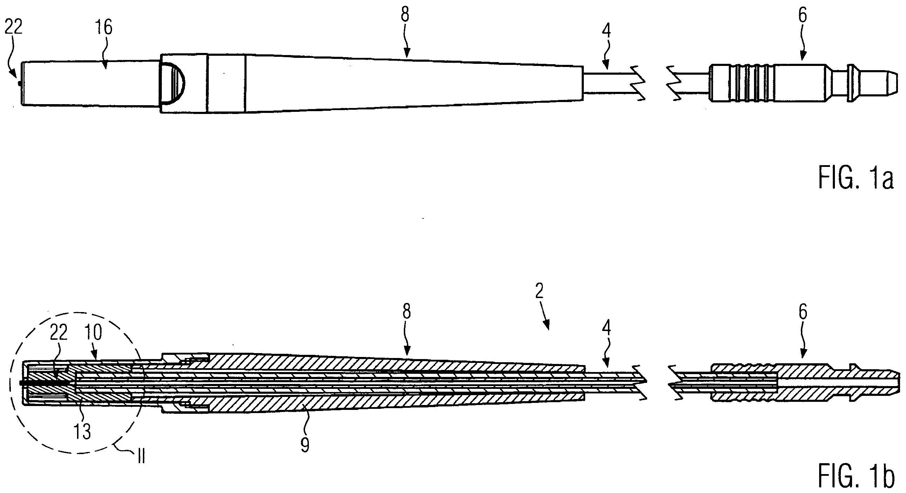

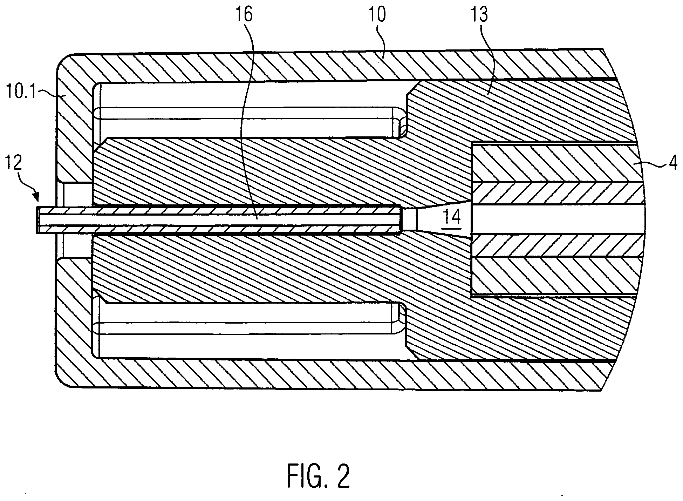

Die Figur 1 zeigt eine Draufsicht auf ein Schlauchelement 2 mit einem flexiblen Fluidschlauch 4, welches einen Innendurchmesser von 0,8 mm und einen Außendurchmesser von 4 mm hat. An dem befestigungsseitigen Ende des Fluidschlauchs 4 befindet sich eine Schlauchkupplung 6, die als Aufsteckkupplung z.B. mit Bajonettsicherung zur Befestigung an einem Auslassstützen an einer nicht dargestellten Pumpe ausgebildet ist. An dem gegenüberliegenden Ende ist der Fluidschlauch 4 mit einem Handstück 8 versehen, welches aus einem Handstückhauptkörper 9 und einer Endkappe 10 gebildet ist, die endseitig unter Einschluss eines einen Düsenkörper 12 haltenden Adapterstücks 13 dem Handstückhauptkörper 9 verbunden ist. Dazu weist der Handstückhauptkörper 9 stirnseitig ein Außengewinde auf, auf welches die Endkappe 10 aufgeschraubt ist. Wie Figur 2 verdeutlicht, hat die Endkappe 10 eine stirnseitige Endfläche 10.1, die stirnseitig gegen das Adapterstück 13 anliegt und dieses in dem Handstück 8 fixiert und eine von dem Düsenkörper 12 durchragte mittlere Bohrung aufweist, die das vordere Ende des Düsenkörpers 12 mit hinreichend Spiel umgibt. An den gegenüberliegenden Enden ist die Schlauchkupplung 6 mit dem Fluidschlauch 4 verklebt.

Das Handstück 8 bildet einen Zwischenkanal aus, der vorliegend als konischer Übergangskanal in dem Adapterstück 13 ausgebildet ist. Das schlauchseitige Ende dieses Zwischenkanals 14 hat einen größeren Durchmesser als der Strömungsdurchmesser des Fluidschlauchs 4. Stromabwärts des Zwischenkanals 14 hat das Adapterstück 13 eine an den Außendurchmesser des Fluidschlauchs 4 angepasste Bohrung, in welcher der Fluidschlauch 4 mit dem Adapterstück 13 verklebt ist. Der Handstückhauptkörper 9 hat eine durchgehende Aufnahmebohrung für den Fluidschlauch 4, in welche der Fluidschlauch 4 mit Spiel gehalten ist und die so ausgebildet ist, dass sich der Fluidschlauch 4 leicht durch diese Bohrung hindurch schieben lässt. Das stromabwärtige Ende des Zwischenkanals 14 ist zylindrisch ausgeformt. Dort geht der Zwischenkanal 14 in einen Strömungsdurchgang 16 über, der durch den Düsenkörper 12 gebildet ist und zu einer Düsenöffnung 18 führt, die jeweils in den nachstehenden Figuren dargestellt und an einem Düsenblech 20 mittels Laserschneiden ausgespart ist. Der metallische Düsenkörper 12 ist in eine mit ihrem Innenumfang an den Außenumfang des Düsenkörpers 12 angepasste Bohrung des Adapterstücks 13 eingesetzt. In dieser Bohrung ist der Düsenkörper 12 mit dem Adapterstück 13 verklebt. Der Strömungsdurchgang 16 hat einen Strömungsquerschnitt, der erheblich kleiner als der stromabwärtige Durchmesser des Zwischenkanals 14 ist. So wird sicher verhindert, dass eventuell beim Fügen aus dem Spalt zwischen den beiden Umfangsflächen von Düsenkörper 12 und Adapterstück 13 herausgedrückter Kleber den Strömungsdurchgang 16 eingangsseitig verlegt. Das Adapterstück 13 ist üblicherweise aus Kunststoff ausgebildet. Die Endkappe 10 kann aus Kunststoff oder Metall ausgeformt sein. Auch das proximale Ende des Fluidschlauchs 4 ist mit der Schlauchkupplung 6 verklebt.

Zum Herstellen des in Figuren 1 und 2 gezeigten Ausführungsbeispiels wird üblicherweise der Fluidschlauch 4 durch das Handstück 8 hindurch geschoben, sodass das freie Ende des Schlauchstücks den Handstückhauptkörper 9 überragt. Ein durch Verkleben von Adapterstück 13 und Düsenkörper 12 vorbereitetes Zwischenerzeugnis wird dann endseitig auf der Fluidschlauch 4 aufgeschoben und mit dem Fluidschlauch 4 verklebt. Danach wird der Fluidschlauch 4 in dem Handstückhauptkörper 9 zurückgezogen, um das Adapterstück 13 stirnseitig gegen den Handstückhauptkörper 9 anzulegen und diese Position durch Aufschrauben der Endkappe 10 zu sichern.

Wie bereits erwähnt, wird das Düsenblech 20 im Grunde mit kreisförmiger Grundfläche aus einer Blech-Halbzeugplatte mittels Laserschneiden ausgeschnitten. Es verbleiben indes radiale Stege, die auch das mit der Düsenöffnung vorbereitete Düsenblech an der größeren Halbzeugplatte halten. So kann das relativ kleine Düsenblech 20 mit der Halbzeugplatte positioniert werden. Danach wird das Düsenblech 20 an den Düsenkörper 12 angeschweißt, gleichzeitig werden die radialen Stege getrennt, sodass das Düsenblech 20 mit kreisrunder Außenumfangsfläche vorliegt und bündig und absatzfrei die zylindrische Außenumfangsfläche des Düsenkörpers 12 fortsetzt. Damit ist ein mit Bezugszeichen 22 gekennzeichnetes Düsenelement geschaffen.

Die nachstehend erläuterten Figuren zeigen Ausführungsbeispiele der Düsenkörper bzw. der Düsenplatten.

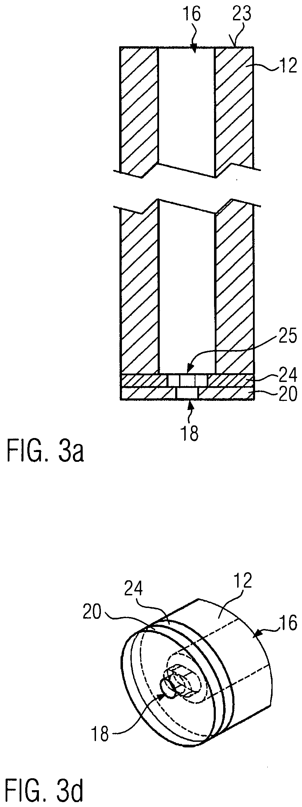

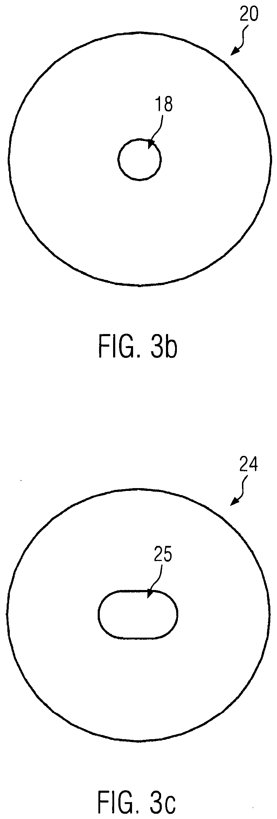

Die Figuren 3a bis 3d zeigen ein erstes Ausführungsbeispiel mit einem zylindrischen Düsenkörper 12 mit dem Strömungsdurchgang 16, der vorliegend einen Durchmesser von 0,3 ± 0,05 mm hat und kreisrund ausgebildet ist. Eingangsseitig bildet der Düsenkörper 12 eine Anlagefläche 23 zur Anlage gegen eine durch das Adapterstück 13 gebildete Ringfläche aus. Das gegenüberliegende Ende des Düsenkörpers 12 ist durch zwei Stahlplatten mit einer Stärke von jeweils 0,7 mm belegt. Dabei ist ein erstes mit Bezugszeichen 24 gekennzeichnetes Blendenblech stirnseitig mit dem Düsenkörper 12 verschweißt. Das Blendenblech 24 hat ein Langloch 25 mit einer Breite von 0,13 mm und einer Länge von 0,21 mm. Die Angaben enthalten Fertigungstoleranzen von 0,005 mm und können um ± 0,03 mm variieren, ohne von dem hier vorgestellten Konzept abzuweichen. Das mit Bezugszeichen 20 gekennzeichnete Düsenblech hat eine kreisförmige zylindrische Düsenöffnung 18 mit einem Durchmesser von 0,114 mm. Diese Düsenöffnung 18 ist konzentrisch zu dem Mittelpunkt des Langloches 25 angeordnet und ausgebildet. Die beiden Öffnungen 18, 25 haben sich parallel zueinander erstreckende Innenwände, die rechtwinklig zu den Vorder- bzw. Rückflächen der jeweiligen Bleche ausgebildet und ausgerichtet sind. So ergeben sich Absätze einerseits zwischen dem Außenumfang des Strömungsdurchgangs 16 und der mit Bezugszeichen 25 gekennzeichneten Blendenöffnung einerseits und dieser Blendenöffnung 25 und der Düsenöffnung 18. Auch das Düsenblech 20 ist mittels Laserschweißen mit dem darunterliegenden Blendenblech verbunden. Das Schweißen erfolgt jeweils stirnseitig. Die Schweißnaht ist durchgeschweißt.

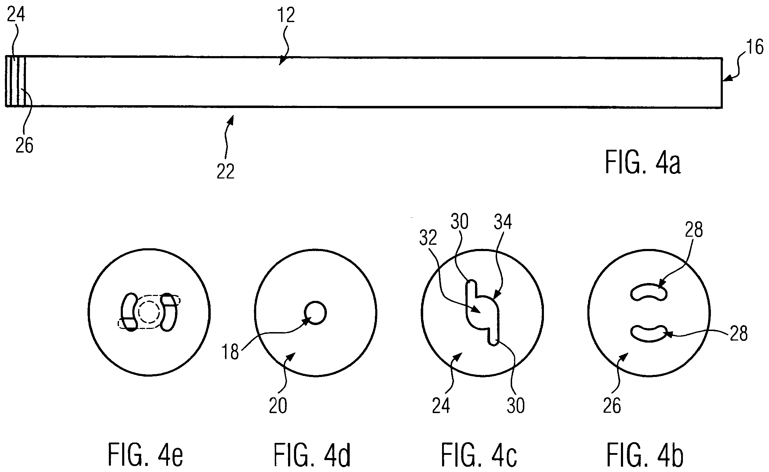

Die Figuren 4a bis 4e verdeutlichen eine zweites Ausführungsbeispiel mit einem Düsenblech 20 (Figur 4d), welches eine kreisrunde Düsenöffnung 18 hat, dem zwei Blendenbleche 24 (Figur 4c) und 26 (Figur 4b) in Strömungsrichtung vorgelagert sind. Dabei ist das in Figur 4b gezeigte erste Blendenblech 26 unmittelbar mit dem Düsenkörper 12 verschweißt. Auf dieses erste Blendenblech 26 ist das zweite Blendenblech 24 aufgeschweißt. Auf dieses wiederum ist das Düsenblech 20 aufgeschweißt. Die Figur 4e zeigt eine Draufsicht auf das erste Blendenblech 26 durch den Strömungsdurchgang 16. Wie ersichtlich fluchten die beiden an dem ersten Blendenblech 26 vorgesehenen, ringsegmentförmigen Ausnehmungen 28 mit schlitzförmigen Verlängerungen 30, die von einer zentralen Bohrung 32 einer Blendenöffnung 34 abgehen, die in dem zweiten Blendenblech 24 ausgespart ist. Diese zentrale Bohrung 32 wiederum fluchtet mit der Düsenöffnung 18. Die beiden Blendenbleche 24, 26 haben eine Stärke von 0,1 mm. Das Düsenblech 20 hat eine Stärke von 0,07 mm.

Durch diese Ausgestaltung wird der durch den Strömungskanal 16 hindurch geleiteten Strömung am Ende ein Drall aufgeprägt, sodass der Düsenstrahl mit einer rotatorischen Geschwindigkeitskomponente an die Umgebung abgegeben wird.

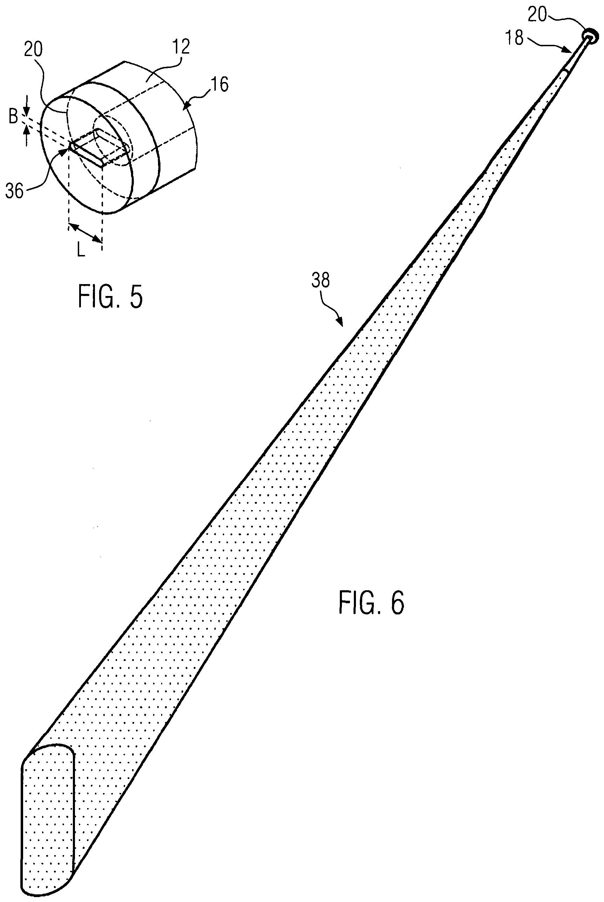

Die Figur 5 verdeutlicht ein weiteres Ausführungsbeispiel eines Düsenblechs 20. Dieses ist mit einer Breitschlitzdüse 36 versehen, bei der die Düsenöffnung 18 zwei gradlinig verlaufende Hauptseitenwände, die sich parallel hierzu erstrecken, und konkav geformte Stirnwände hat. Die Stirnwände sind vorliegend mit einem Radius entsprechend der halben Breite der Breitschlitzdüse 36 ausgebildet. Die Breite B der Düsenöffnung 18 beträgt 0,05 mm. Die Länge L beträgt 0,256 mm. Auch hier können die genannten Maße um +/-25% variieren, ohne dass von dem hier diskutierten Konzept abgewichen wird. Das Düsenblech 20 hat eine Stärke von 0,20 mm. Sämtliche Ausnehmungen und Konturen sind lasergeschnitten. Das bevorzugte Verhältnis von Länge L zu Breite B liegt bei zwischen 4,3 und 6,5, vorliegend bei 5,1.

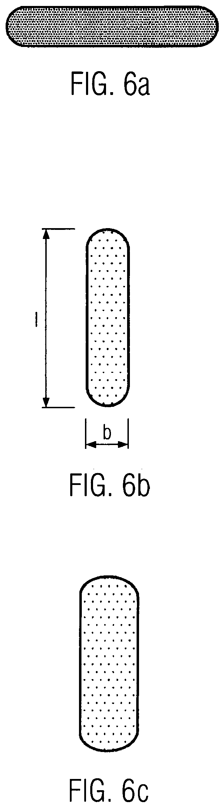

Die Figur 6 verdeutlicht schematisch einen Flachstrahl 38, der durch die unter Bezugnahme auf die Figur 5 diskutierte Ausführungsform der Breitschlitzdüse 36 an die Umgebung abgegeben wird. Dabei verdeutlichen die Figuren 6a und 6b Schnittansichten des Flachstrahls einerseits auf Höhe der Breitschlitzdüse 36 (Figur 6a) und andererseits in einem Abstand von 80 mm von der Breitschlitzdüse. Die Ansicht ist schematisch, wobei der Querschnitt des Flachstrahls auf Höhe der Breitschlitzdüse 36 in Figur 6a in einem 40-fach größeren Maßstab als der Maßstab nach Figur 6b gezeichnet wurde. Bereits Figur 6 verdeutlicht, dass sich der an die Umgebung abgegebene Flachstrahl 38 in der Umgebung umorientiert, sodass bei einer unterstellt horizontalen Ausrichtung des Flachstrahls 38 auf der Höhe der Breitschlitzdüse 36 dieser in einem Abstand von 80 mm eine Längserstreckung in vertikaler Richtung hat. Die Breitenausrichtung des Flachstrahles 38 ist damit um 90° gedreht, wie dies die Figuren 6a und 6b verdeutlichen. Die Querschnittsform nach Figur 6b ergibt sich für einen Flachstrahl, der mit einem Winkel von 90° auf die zu behandelnde Oberfläche auftritt. Üblicherweise wird der Flachstrahl indes relativ zu dieser Oberfläche geneigt. Figur 6c verdeutlicht schematisch diejenige Fläche des Flachstrahls 38, der sich für eine wirksame Reinigung der Oberfläche nutzen lässt, bei einer Ausrichtung des Flachstrahls in einem 45° Winkel relativ zu der zu behandelnden Oberfläche.

Die Figur 6b lässt erkennen, dass der sich entwickelnde Flachstrahl ein gutes Breiten-zu-Längen-Verhältnis l/b hat. Ein Flachstrahl im Sinne der Erfindung kann auch in dem Arbeitsabstand ein Strahlstoßdruckprofil mit konvexen Rändern innerhalb des wirksamen Druckprofils haben. Der auf die zu behandelnde Oberfläche mit einem Arbeitsabstand von 80 mm auftreffende Flachstrahl 38 hat innerhalb der in Figur 6b gezeigten wirksamen Fläche einen Strahlstoßdruck von zumindest 1.000 Pa, eine Länge von 9 mm und eine Breite von 2 mm. Außerhalb dieser Abmessungen führt zwar der Flachstrahl 28 Flüssigkeit mit sich. Der Strahlstoßdruck dieser äußeren Bereiche des Flachstrahls zeigt indes auf der zu behandelnden Oberfläche nicht die gewünschte Wirkung und wird den Maßen des hier interessierenden Flachstrahls nicht zugerechnet.

### Bezugszeichenliste

- 2: Schlauchelement
- 4: Fluidschlauch
- 6: Schlauchkupplung
- 8: Handstück
- 9: Handstückhauptkörper
- 10: Endkappe
- 10.1: Stirnseitige Endfläche
- 12: Düsenkörper
- 13: Adapterstück
- 14: Zwischenkanal
- 16: Strömungsdurchgang
- 18: Düsenöffnung
- 20: Düsenblech
- 22: Düsenelement
- 23: Anlagefläche
- 24: Blendenblech
- 25: Langloch
- 26: Blendenblech
- 28: ringsegmentförmige Ausnehmungen
- 30: schlitzförmige Verlängerungen
- 32: Bohrung
- 34: Blendenöffnung
- 36: Breitschlitzdüse
- 38: Flachstrahl
- 40: Hüllfläche

## Patentansprüche

1. Düsenelement (22) zum Einbringen in ein Handstück (8) zum Aufstrahlen eines Wasserstrahls mit einem Wasserdruck zwischen 50 und 200 bar, das einen länglichen zylindrischen Düsenkörper (12), der einen Strömungsdurchgang für den Wasserstrahl ausbildet, und zumindest eine einen Düsenstrahl an die Umgebung abgebende Düsenöffnung (18) hat, deren kleinste Abmessung nicht mehr als 0,15 mm ist, **dadurch gekennzeichnet, dass** im Falle einer kreisrunden Düsenöffnung (18) deren Durchmesser mindestens 0,09 mm und höchstens 0,12 mm beträgt, dass die Düsenöffnung (18) so ausgebildet ist, dass sich bei einem Wasserdruck von 130 bar in einem Arbeitsabstand von 80 mm zwischen der Düsenöffnung (18) und der zu behandelnden Oberfläche auf der zu behandelnden Oberfläche ein Flachstrahl ergibt, dass der Düsenkörper (12) mit einer Länge von zwischen 1 und 30 mm ausgebildet ist, dass der Strömungsdurchgang einen runden Querschnitt und einem Durchmesser von zwischen 0,15 mm und 0,6 mm hat und dass der Düsenkörper (12) aus Stahl gebildet ist und mit einem die Düsenöffnung (18) ausbildenden Düsenblech (20) verschweißt ist.

2. Düsenelement (22) nach Anspruch 1, **dadurch gekennzeichnet, dass** die Düsenöffnung (18) von einer Innenseite bis zu einer Außenseite des Düsenblechs (20) zylindrisch ausgebildet ist.

3. Düsenelement (22) nach Anspruch 1 oder 2, **dadurch gekennzeichnet, dass** die Düsenöffnung durch eine der Breitschlitzdüse (36) gebildet ist, deren Breite (B) zwischen 0,035 und 0,06, bevorzugt zwischen 0,04 und 0,055 mm liegt.

4. Düsenelement (22) nach Anspruch 3, **dadurch gekennzeichnet, dass** die Breitschlitzdüse (36) geradlinig verlaufende Hauptseitenwände und mit einem zumindest der halben Breite (B) entsprechenden Radius, der und nicht größer als 0,15 mm ist, konkav gekrümmte Stirnwände aufweist.

5. Düsenelement (22) nach einem der Ansprüche 1 bis 4, **dadurch gekennzeichnet, dass** die Düsenöffnung (18) mit einem mittleren Durchmesser von zwischen 0,09 mm und 0,12 mm oval ausgebildet ist.

6. Düsenelement (22) nach einem der Ansprüche 1 bis 5, **dadurch gekennzeichnet, dass** dem Düsenblech in Strömungsrichtung zumindest ein Blendenblech vorgelagert ist, dessen Blendenöffnung größer ist als die Düsenöffnung.

7. Düsenelement (22) nach einem der Ansprüche 1 bis 6, **dadurch gekennzeichnet, dass** dem Düsenblech in Strömungsrichtung zumindest zwei Blendenbleche vorgelagert sind, die voneinander abweichende Blendenöffnungen haben, die jeweils nicht-rotationssymmetrisch zu einer Mittelängsachse der Düsenöffnung jedoch punktsymmetrisch zu dieser Mittellängsachse ausgeformt oder angeordnet sind.

8. Schlauchelement (2) mit einem Fluidschlauch (4), der an einem Eintrittsende mit einem Schlauchkupplung (6) zum Anschluss des Schlauchelementes an eine Pumpe und an seinem Austrittsende ein Handstück (8) aufweist, das endseitig mit einem Düsenelement (22) nach einem der Ansprüche 1 bis 7 verbunden ist.

9. Handstück (8) für ein Schlauchelement nach Anspruch 8 mit einem Handstückhauptkörper (9) der eine längliche Bohrung zur Aufnahme des Fluidschlauches (4) aufweist und endseitig eine Endkappe (10) trägt, die zwischen sich und dem Handstückhauptkörper (9) ein Adapterstück (13) einschließt, das mit einem Düsenelement (22) nach einem der Ansprüche 1 bis 7 verbunden ist.

10. Handstück nach Anspruch 9, dass das Düsenelement (22) und der Fluidschlauch (4) mit dem Adapterstück (13) dicht verbunden, insbesondere verklebt sind.

11. Verfahren zum Herstellen eines Düsenelementes (22) nach einem der Ansprüche 1 bis 7, bei dem mittels eines Lasers zumindest eine Düsenöffnung (18) an einem Düsenblech (20) ausgebildet wird und dabei das Düsenblech (20) aus einer Düsenblechplatte geschnitten wird, wobei Verbindungsstege zwischen dem Düsenblech (20) und der Düsenblechplatte verbleiben, so dass das Düsenblech (20) über die Düsenblechplatte gehandhabt und positioniert werden kann und danach das Düsenblech (20) mit dem Düsenkörper (12) verschweißt wird und dabei die verbleibenden Verbindungsstege durchtrennt werden.

## Claims

1. Nozzle element (22) to be introduced into a handpiece (8) for emitting a water jet at a water pressure between 50 and 200 bar, which has a cylindrical nozzle body (12) forming a flow passage for the water jet, and at least one nozzle opening (18) emitting a jet stream to the surrounding area whose smallest dimension is not more than 0.15 mm
**characterized in that**
in case of a circular nozzle opening (18) with a diameter of at least 0.09 mm and maximum 0.12 mm the nozzle opening (18) is formed such that a fan jet results on the surface to be treated between the nozzle opening (18) and the surface to be treated at a water pressure of 130 bar at a working distance of 80 mm, that the nozzle body (12) has a length of between 1 and 30 mm, that the flow passage has a circular cross section and a diameter of between 0.15 mm and 0.6 mm and that the nozzle body (12) is made of steel and welded to a nozzle plate (20) forming the nozzle opening (18).

2. Nozzle element (22) according to claim 1, **characterized in that** the nozzle opening (18) is embodied to be cylindrical from an inner side to an outer side of the nozzle plate (20).

3. Nozzle element (22) according to claim 1 or 2, **characterized in that** the nozzle opening (18) is a sheet nozzle (36) having a width (B) of between 0.035 and 0.06, preferably between 0.04 and 0.055 mm.

4. Nozzle element (22) according to claim 3, **characterized in that** the sheet nozzle (36) comprises straight main side walls and has a radius corresponding to at least half the width (B) which is not larger than 0.15 mm and comprises concavely bent front walls.

5. Nozzle element (22) according to one of claims 1 to 4, **characterized in that** the nozzle opening (18) is embodied to be circular with a diameter of between 0.09 mm and 0.12 mm, or to be oval with a corresponding mean diameter.

6. Nozzle element (22) according to one of claims 1 to 5, **characterized in that** at least one shield plate is preceding the nozzle plate in the direction of flow whose shield opening is larger than the nozzle opening.

7. Nozzle element (22) according to one of claims 1 to 6, **characterized in that** at least two shield sheets are preceding the nozzle plate in the direction of flow, which have differing shield openings, which are each formed or arranged not rotationally symmetrically to a longitudinal central axis of the nozzle opening, however point-symmetrically to this longitudinal central axis.

8. Hose element (2) with a fluid hose (4) which has, at an inlet end, a hose coupling (6) for connecting the hose element to a pump and, at its outlet end, a handpiece (8) which is connected to a nozzle element (22) according to one of claims 1 to 7 at the end side.

9. Handpiece (8) for a hose element according to claim 8 with a handpiece main body (9) comprising an oblong bore for receiving the fluid hose (4) and carrying an end cap (10) at the end side which encloses an adapter piece (13) between itself and the handpiece main body (9) which is connected to a nozzle element (22) according to one of claims 1 to 7.

10. Handpiece according to claim 9, that the nozzle element (22) and the fluid hose (4) are tightly connected to the adapter piece (13), in particular glued together.

11. Method of manufacturing a nozzle element (22) according to one of claims 1 to 7, embodying at least one nozzle opening (18) at a nozzle plate (20) by means of a laser, thereby cutting a nozzle plate (20) out of the nozzle sheet plate, leaving connection webs between the nozzle plate (20) and the nozzle sheet plate for positioning the nozzle plate (20) via the nozzle sheet plate and then welding the nozzle plate (20) to the nozzle body (12) thereby cutting the remaining connection webs.

## Revendications

1. Élément formant buse (22) destiné à être inséré dans une pièce à main (8) pour la projection d'un jet d'eau sous une pression d'eau comprise entre 50 et 200 bars, comportant un corps de buse cylindrique allongé (12) formant un passage d'écoulement pour le jet d'eau et au moins une ouverture de buse (18) délivrant un jet de buse dans l'environnement, dont la plus petite dimension n'est pas supérieure à 0,15 mm,
**caractérisé en ce que**
dans le cas d'un orifice de buse circulaire (18), dont le diamètre est d'au moins 0,09 mm et d'au plus 0,12 mm, l'orifice de buse (18) est conçu de sorte que, pour une pression d'eau de 130 bars, un jet plat est produit sur la surface à traiter à une distance de travail de 80 mm entre l'orifice de buse (18) et la surface à traiter, que le corps de buse (12) présente une longueur comprise entre 1 et 30 mm, que le passage d'écoulement présente une section transversale ronde et un diamètre compris entre 0,15 mm et 0,6 mm, et que le corps de buse (12) est constitué d'acier et qu'il est soudé à une tôle de buse (20) formant l'ouverture de buse (18).

2. Élément formant buse (22) selon la revendication 1, **caractérisé en ce que** l'ouverture de buse (18) est cylindrique d'un côté intérieur vers un côté extérieur de la tôle de buse (20).

3. Élément formant buse (22) selon la revendication 1 ou 2, **caractérisé en ce que** l'ouverture de buse est formée par l'une des buses à large fente (36), dont la largeur (B) est comprise entre 0,035 et 0,06, de préférence entre 0,04 et 0,055 mm.

4. Élément formant buse (22) selon la revendication 3, **caractérisé en ce que** les parois latérales principales rectilignes de la buse à large fente (36), d'un rayon qui correspond à au moins la moitié de la largeur (B) et non supérieur à 0,15 mm, présentant des parois d'extrémité incurvées de manière concave.

5. Élément formant buse (22) selon l'une des revendications 1 à 4, **caractérisé en ce que** l'ouverture de buse (18) est de forme ovale et présente un diamètre moyen compris entre 0,09 mm et 0,12 mm.

6. Élément formant buse (22) selon l'une des revendications 1 à 5, **caractérisé en ce qu'**au moins une tôle de protection, dont l'ouverture de l'orifice est plus grande que l'ouverture de la buse, est disposée en amont de la tôle de buse dans le sens de l'écoulement.

7. Élément formant buse (22) selon l'une des revendications 1 à 6, **caractérisé en ce qu'**au moins deux tôles de protection sont montées en amont de la tôle de buse dans le sens de l'écoulement, lesquelles tôles de protection présentent des ouvertures de protection différentes les unes des autres, lesquelles ouvertures de protection sont chacune formées ou disposées sans symétrie axiale par rapport à un axe longitudinal central de l'ouverture de buse mais disposées en symétrie centrale par rapport à cet axe longitudinal central.

8. Élément tuyau (2) ayant un tuyau de liquide (4) qui présente à une extrémité d'entrée un raccord de tuyau (6) pour le raccordement de l'élément tuyau à une pompe et à son extrémité de sortie une pièce à main (8) dont l'extrémité est reliée à un élément formant buse (22) selon l'une des revendications 1 à 7.

9. Pièce à main (8) pour un élément tuyau selon la revendication 8, comprenant un corps principal de pièce à main (9) ayant un alésage allongé pour recevoir le tuyau de liquide (4) et portant à son extrémité un bouchon d'extrémité (10) qui inclut entre lui et le corps principal de pièce à main (9), un adaptateur (13) relié à un élément formant buse (22) selon l'une des revendications 1 à 7.

10. Pièce à main selon la revendication 9, l'élément formant buse (22) et le tuyau de liquide (4) étant raccordés de manière étanche, plus spécifiquement collés, à l'adaptateur (13).

11. Procédé de fabrication d'un élément formant buse (22) selon l'une des revendications 1 à 7, dans lequel au moins une ouverture de buse (18) est formée sur une tôle de buse (20) au moyen d'un laser, et dans lequel la tôle de buse (20) est découpée à partir d'une plaque de tôle de buse, des ponts de liaison demeurant entre la tôle de buse (20) et la plaque de tôle de buse de sorte que la tôle de buse (20) peut être manipulée et positionnée sur la plaque de tôle de buse et qu'ensuite la tôle de buse (20) peut être soudée au corps de la buse (12), séparant ainsi les ponts de liaison restants.
